# EUROPEAN PATENT APPLICATION

(11) **EP 4 793 014 A1**
(43) Date of publication of application: **19.08.2026**
(21) Application number: 24877587.6
(22) Date of filing: 14.10.2024
(51) Int. Cl.: B29B 17/04, B09B 3/60, B29B 17/02, B09B 101/75

(54) **METHOD FOR IMPROVING QUALITY OF RECYCLED PLASTIC USING MICROORGANISMS**

(30) Priority: 13.10.2023 KR 20230136822
(71) Applicant: Repla Inc., Gyeongsan-si, Gyeongsangbuk-do 38541 (KR)
(72) Inventor: SUH, Dong Eun, Yongin-si Gyeonggi-do 17103 (KR); KIM, Hong Rae, Suwon-si Gyeonggi-do 16678 (KR); KOH, Hye Yeon, Suwon-si Gyeonggi-do 16668 (KR)
(74) Representative: Schiweck Weinzierl Koch Patentanwälte Partnerschaft mbB
(86) International application number: PCT/KR2024/015536
(87) International publication number: WO 2025/080052

(57) **Abstract**

The present invention relates to a method for improving quality such as purity and physical properties of plastics regenerated from waste by treating polymer waste such as plastics with microorganisms having plastic degradation activity. By using the method, foreign substances contained in the polymer waste can be removed, and the melt flow rate, tensile strength, and flexural strength of the recycled plastic can be improved.

## Description

### [Technical Field]

The present invention relates to a method of improving the quality, such as purity and physical properties, of recycled plastic by treating the recycled plastic with a microorganism having plastic-degrading activity.

### [Background Art]

Plastic refers to a solid material formed into a useful shape using a polymeric material as a main raw material, and is also referred to as a synthetic resin. Since plastic is a type of polymer in which numerous monomers having the same chemical structure are linked to form a large molecule, it can be manufactured to have various forms and characteristics depending on its chemical composition.

The use of plastics has rapidly increased due to their light weight, high physicochemical durability, high processability, and very low cost. The annual global production of plastics, which was 2 million tons in 1950, reached nearly 370 million tons as of 2019. In addition, the spread and persistence of COVID-19 have led to a surge in the consumption of personal protective equipment, such as disposable masks and gloves, which are primarily made of plastic; an increase in the use of disposable tableware due to the rise in food takeout and delivery services; and an increase in the use of plastic packaging containers driven by the expansion of online shopping. Consequently, plastic consumption is expected to continue to increase in the future (Reference 1).

However, only about 13% of the generated plastic is utilized through material recycling. Material recycling refers to a method of recycling in which used plastics are reprocessed into new plastic products, and is the most environmentally friendly recycling method. The difficulty in increasing the material recycling rate of plastics lies in the low quality of the recycled plastics produced through recycling. The primary causes of quality deterioration in recycled plastics include contamination by foreign substances during the recycling sorting process and the incorporation of additives during the manufacturing process. As a result, recycled plastics are traded at lower prices than virgin plastics, and their applications are limited, as they cannot be used in products requiring high-quality plastics, such as electronic devices.

Specifically, the causes of quality deterioration during the plastic recycling process include: 1) a decrease in the purity of the plastic due to the mixing of foreign substances; 2) additives mixed in during the plastic manufacturing process; and 3) a degradation of physical properties due to natural oxidation during the use of the plastic.

Meanwhile, microorganisms are attracting attention as a means for treating and recycling plastic waste. Microorganisms and enzymes are environmentally friendly and energy-efficient because they can degrade plastics under ambient temperature and pressure conditions. Research on microorganisms capable of degrading plastics and enzymes produced by these microorganisms is actively being conducted (Reference 2). However, research utilizing microorganisms has focused only on the degradation of plastics, and prior patent documents have likewise concentrated on the discovery of microorganisms or plastic-degrading enzymes (KR10-22461894B1; WO2023-027529A1; and KR10-2126889B1). Nevertheless, to enhance plastic recycling from an industrial perspective, there is a need not only for plastic degradation technologies but also for technologies capable of improving the quality of recycled plastics.

### [Disclosure]

### [Technical Problem]

Under these circumstances, the present inventors have studied a method of improving the quality of polymer waste, such as plastic, and have devised the present invention. Specifically, the present inventors have confirmed that when a plastic-degrading microorganism is allowed to react with plastic waste and the waste is then recycled, the physical properties of the recycled plastic are significantly improved.

Therefore, an object of the present invention is to develop a technology for improving the quality of recycled plastics by utilizing microorganisms.

### [Technical Solution]

In order to achieve the above object,
the present invention provides a method of improving the purity of polymer waste, including
bringing a microorganism having plastic-degrading activity, a culture broth of the microorganism, or a plastic-degrading enzyme derived from the microorganism into contact with polymer waste,
wherein the polymer is a polymer synthesized from petrochemicals or an organic polymer.

In addition, the present invention provides a method of improving the physical properties of a product recycled from polymer waste, including
bringing a microorganism having plastic-degrading activity, a culture broth of the microorganism, or a plastic-degrading enzyme derived from the microorganism into contact with polymer waste,
wherein the polymer is a polymer synthesized from petrochemicals or an organic polymer.

The product may be a plastic.

In the present invention, the polymer synthesized from petrochemicals may be one or more plastics (synthetic resins) selected from the group consisting of polyethylene terephthalate (PET), polyvinyl chloride (PVC), polystyrene (PS), polypropylene (PP), polyurethane (PU), and polyethylene (PE). In addition, it may be a polymeric material synthesized from various hydrocarbon components derived from a distillation process of crude oil.

In an embodiment of the present invention, polyethylene was used as an example of the polymer synthesized from petrochemicals.

Polyethylene terephthalate has high transparency and is tasteless and odorless, thus accounting for most of the plastic beverage bottles distributed on the market. Polyvinyl chloride is a thermoplastic plastic that is strong, rigid or flexible, and resistant to wear. Polyvinyl chloride is used in artificial leather, packaging materials, pipes, electrical insulators, and the like, and is the plastic that has been popular for the longest time under the name of "vinyl."

Polystyrene is a thermoplastic plastic that is lightweight, tasteless, and odorless, and is used in household goods, toys, electrical insulators, radios, television cases, packaging materials, and the like. Polypropylene is polymerized from propylene obtained from petroleum and is widely used for bottles, containers, and the like.

Polyethylene is a thermoplastic plastic that is lightweight and flexible, and is a general-purpose plastic used for everything from industrial materials to daily necessities. High-density polyethylene (HDPE) has strong impact resistance and good cold resistance, and thus is mainly used to make shopping bags or pipes. Since low-density polyethylene (LDPE) has branches in its polymer, it has a lower density than linear HDPE, and is easy to process due to its good elasticity.

In the present invention, the organic polymer may be one or more selected from the group consisting of fibers, synthetic resins, synthetic rubber, and polysaccharides.

In the present invention, the "method of improving the purity of polymer waste during a recycling process" refers to a method of reducing the content of foreign substances during the sorting process of polymer waste recycling. Generally, the sorting process for polymer waste recycling involves removing substances other than the target material to be recycled. For example, in the case of recycled PP plastics, any plastic other than PP is considered a foreign substance.

However, conventional physical sorting methods struggle to distinguish between plastics with similar properties, which limits the extent to which the content of foreign substances can be reduced. Furthermore, additives cannot be removed using physical methods because they are integrally mixed into the plastics. Meanwhile, chemical sorting methods require complex and costly processes to remove these contaminants and additives.

In contrast, the present invention utilizes microorganisms and enzymes to improve plastic quality by: 1) increasing plastic purity through the selective degradation of contaminant materials; 2) selectively degrading additives using microorganisms; and 3) selectively degrading regions that have deteriorated due to factors like natural oxidation during the plastic's lifespan. While these effects are particularly prominent in recycled plastics, they can be applied to all plastics, including virgin resins.

In the present invention, "plastic degradation" refers to either degrading the polymeric materials constituting the plastic into low-molecular-weight intermediates or intermediates capable of being metabolized through microbial metabolic pathways, or oxidizing the polymeric materials.

In the present invention, "recycled plastic" refers to plastic that is recovered after its use as a product and is subjected to material recycling, or plastic remanufactured into a product through material recycling. Additionally, "plastic waste" refers to plastic discarded after use, which is typically processed into flakes for recycling.

In the present invention, the "quality of plastic" refers to its properties, such as purity, thermal properties, mechanical properties, and melt flow rate. The quality is considered high when these properties closely resemble those of standard virgin plastics used in the industry. The mechanical properties include tensile strength, impact strength, and flexural modulus.

In the present invention, the contacting step may be performed for 0.1 hours to 60 days, and preferably for 1 day to 30 days, but this may vary depending on the growth rate and culture conditions of the plastic-degrading microorganism.

In the present invention, the microorganism having plastic-degrading activity may be selected from the group consisting of a *Bacillus toyonensis* reborn strain deposited under Accession No. KACC 81043BP, a *Pseudomonas aeruginosa* repla1 strain deposited under Accession No. KACC 81230BP, an *Acinetobacter guillouiae* repla2 strain deposited under Accession No. KACC 81234BP, and a *Rhodococcus yunnanensis* repla5 strain deposited under Accession No. KACC 81286BP.

The technology for improving the quality of recycled plastics according to the present invention may be utilized in various ways. First, it may be used for the purpose of improving the purity of recycled plastic. It may be used to remove foreign substances and additives in the recycled plastic, leaving the plastic in a pure form. Second, it can be used for the purpose of improving the physical properties of recycled plastic. The purpose of using recycled plastics varies depending on their physical properties, and recycled plastics with improved physical properties may be utilized in high value-added products. Generally, high physical properties mean that the mechanical properties of the recycled plastic have been improved. The physical properties of the plastic may be improved by selectively degrading low-molecular-weight plastic materials and selectively degrading regions where natural oxidation has occurred during the use of the plastic, using microorganisms.

### [Advantageous Effects]

By using the method of the present invention, it is possible to effectively improve the quality of recycled plastic, such as purity, melt flow rate, tensile strength, and flexural strength.

### [Description of Drawings]

FIG. 1 shows the results of confirming the degradation rates of virgin plastic and recycled plastic by plastic-degrading microorganisms.
FIG. 2 shows the DSC analysis results of a virgin plastic (a), a recycled plastic (b), and a recycled plastic treated with a microorganism (c).
FIG. 3 shows the results of quantifying the heights of PE and PP peaks in a virgin plastic (PP), a recycled plastic (PP), and a recycled plastic (PP) treated with a microorganism.
FIG. 4 shows the results of quantifying the PE/PP ratio before and after the microorganism treatment of a recycled plastic (PP).
FIG. 5 shows specimens prepared for measuring the physical properties of plastics: (a) a recycled plastic; (b) a recycled plastic treated with *Pseudomonas* sp.; (c) a recycled plastic treated with *Acinetobacter* sp.; and (d) a virgin plastic.

### [Modes of the Invention]

Hereinafter, one or more embodiments will be described in more detail through Examples. However, these Examples are provided to illustrate one or more embodiments, and the scope of the present invention is not limited to these Examples.

### Example 1: Improvement of Purity of Plastic Waste

Generally, it is difficult to sort plastics during the recycling process if their densities and chemical structures are similar. Therefore, the plastics that get mixed in as foreign substances during the plastic recycling process are limited to those having similar properties. For example, polypropylene (PP) mainly contains polyethylene (PE), which has a similar density and chemical structure, as a foreign substance.

Since plastic-degrading microorganisms have selective degradation abilities for different types of plastics, the purity of a plastic may be increased using a strain that lacks the ability to degrade that specific plastic. For example, if a strain that cannot degrade PP but can degrade PE is used, the purity of recycled PP may be improved. Furthermore, since plastics are more difficult to degrade compared to the additives contained in recycled plastics, only the additive materials may be selectively degraded through a mixed culture with microorganisms. Consequently, this leads to an improvement in the purity of the recycled plastic.

The *Pseudomonas aeruginosa* repla1 strain having plastic-degrading activity was cultured in a nutrient medium (Luria-Bertani broth, LB) for 7 days. The culture temperature was 28 °C, and the agitation condition was 130 rpm. After culturing, 1 mL of the culture broth was extracted and centrifuged (13,000 rpm, 5 minutes), after which the supernatant was discarded and only the cells were collected. The cells were washed twice using 0.9% physiological saline to remove all nutritional components, and then used in the experiment.

The composition of the minimal medium used in the experiment was as follows: based on 1 L of distilled water at pH 6.51, 0.7 g of NH₂PO₄, 0.7 g of K₂HPO₄, 0.7 g of MgSO₄·7H₂O, 1.0 g of NH₄NO₃, 0.005 g of NaCl, 0.002 g of FeSO₄·7H₂O, 0.002 g of ZnSO₄·7H₂O, and 0.001 g of MnSO₄·H₂O.

Virgin polypropylene and recycled polypropylene were supplied as carbon sources to the minimal medium and cultured for 7 days, after which the degradation rate by the repla1 strain was determined. The repla1 strain showed a degradation rate of 0.03% for virgin polypropylene, but a degradation rate of 1.02% for recycled polypropylene, exhibiting a significant difference. Since the repla1 strain has a very low degradation ability for polypropylene, it can be considered that the foreign substances and additives in the recycled polypropylene were degraded.

To confirm whether the microorganisms proliferated during the culture period, the viable cell count was measured. Initially, the microorganisms were inoculated at a concentration of about 5.0 x 10⁵ (CFU/mL), and in virgin polypropylene, the microorganisms died, decreasing to 2.37 x 10⁵ CFU/mL. On the other hand, in recycled polypropylene, the microorganisms proliferated by degrading foreign substances, increasing 1000-fold to 5.17 x 10⁸ CFU/mL. Table 1 shows the viable cell counts of microorganisms cultured using virgin polypropylene and recycled polypropylene as carbon sources.

**[Table 1]**

| Strain | Plastic | Initial Inoculation (CFU/mL) | 7-Day Culture (CFU/mL) |
|---|---|---|---|
| repla1 | Virgin plastic (PP) | 6.90 × 10⁵ | 2.37 × 10⁵ |
| | Recycled plastic (PP) | 4.70 × 10⁵ | 5.17 × 10⁸ |

Differential Scanning Calorimetry (DSC) analysis was conducted to determine whether foreign substances in the recycled plastic were removed. Since each type of plastic exhibits a heat rate at its specific temperature, the presence of foreign substances can be identified through DSC analysis.

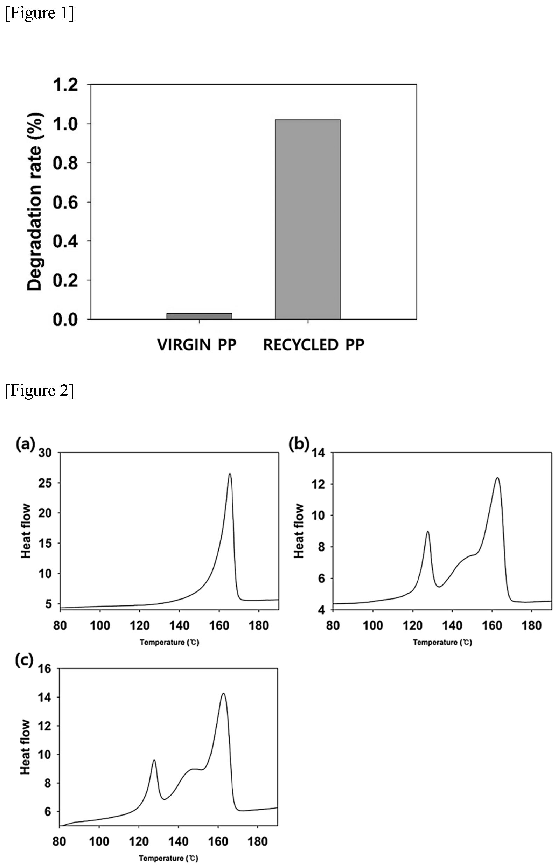

In recycled polypropylene, a peak for polypropylene appears at 162 °C, and a peak for polyethylene, which is often mixed in as a foreign substance, appears at 128 °C. For virgin PP, a peak was observed only at 162 °C (FIG. 2A), while recycled PP (FIG. 2B) and microorganism-treated recycled PP (FIG. 2C) showed an additional peak at 128 °C.

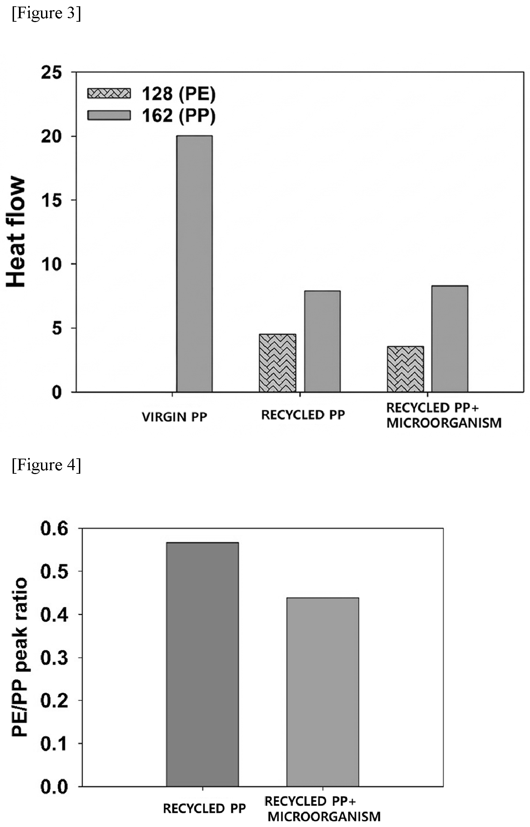

The peaks that appeared at 128 °C and 162 °C for the three samples were plotted as a graph (FIG. 3). From these results, based on the ratio of the peaks at 128 °C and 162 °C, the relative proportion of PE decreased in the microorganism-treated sample (FIG. 4). This indicates that the purity of PP was improved as the microorganism selectively degraded PE, a foreign substance.

Subsequently, pyrolysis-gas chromatography/mass spectrometry (Py-GC/MS) was performed. This is a method of pyrolyzing a sample, separating the products by size through gas chromatography, and then identifying what the substances are. Through this, additives within plastic can be analyzed. In the recycled plastic, two types of antioxidants were identified, and their proportions decreased through microorganism treatment (Table 2).

**[Table 2]**

| Type of Additive | Recycled Plastic | Recycled Plastic + repla1 |
|---|---|---|
| 2,4-Di-tert-butylphenol | 0.3% | 0.2% |
| Phenol, 2,4-bis(1,1-dimethylethyl)-, phosphite (3:1) | 2.3% | 1.9% |

### Example 2: Improvement of Physical Properties of Recycled Plastic

The physical properties of a recycled plastic treated with microorganisms are generally known to have a high correlation with the melt index (MI), and it is known that the lower the melt index, the more improved the physical properties of the plastic (Reference 3). Therefore, the melt flow rate (MFR) of a virgin plastic, a recycled plastic, and a recycled plastic treated with microorganisms was measured. For the measurement conditions, the amount of plastic extruded per unit time under a load of 2.16 kg at 230 °C was measured and then converted into g/10 min, which is the official standard. The microorganism culture was performed utilizing 3 strains of recycled plastic-degrading microorganisms and 1 strain of the genus *Klebsiella* having no recycled plastic-degrading activity. The 3 strains of plastic-degrading microorganisms were the *Pseudomonas aeruginosa* repla1 strain deposited under Accession No. KACC 81230BP, the *Acinetobacter guillouiae* repla2 strain deposited under Accession No. KACC 81234BP, and the *Rhodococcus yunnanensis* repla5 strain deposited under Accession No. KACC 81286BP.

In the results below, the 3 strains, repla1, repla2, and repla5, exhibited degrading activity for the recycled plastic, and an increase in optical density due to microorganism proliferation was also confirmed. On the other hand, no degrading activity of the *Klebsiella* sp. strain was confirmed. Looking at the MFR, it was confirmed that the MFR decreased in the experimental groups cultured with the microorganisms having plastic-degrading activity (Table 3).

**[Table 3]**

| Experimental Group | Recycled Plastic Degradation Rate (%) | Optical Density (OD600) | MFR (g/10 min) |
|---|---|---|---|
| repla1 | 1.02 | 0.316 | 26.8 |
| repla2 | 1.87 | 0.926 | 24.9 |
| repla5 | 0.73 | 0.198 | 25.0 |
| *Klebsiella* sp. | 0.07 | 0.012 | 29.4 |
| Recycled plastic without microorganism treatment | - | - | 29.7 |
| Virgin plastic | - | - | 16.5 |

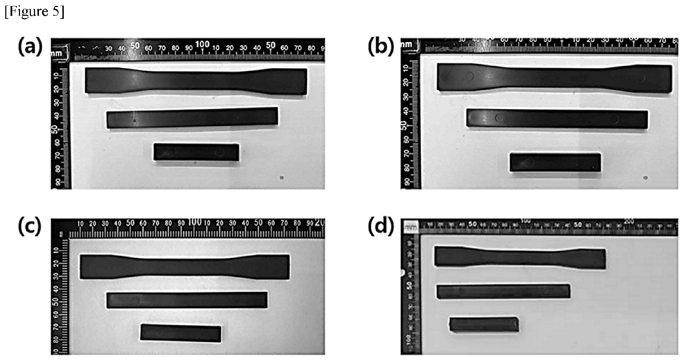

To confirm whether the mechanical properties of the recycled plastic were improved through microorganism treatment, an experiment was conducted as follows. About 7 kg of recycled plastic was allowed to react with plastic-degrading microorganisms in a 100 L capacity microorganism culture vessel, and then extruded and molded to prepare uniform specimens (FIG. 5). The tensile strength and flexural strength of the specimens were measured.

As a result, it was found that the tensile strength and flexural strength of the plastic were improved through microorganism treatment, and the level of improvement was more pronounced as the extent of the MFR decrease became larger. This improvement in physical properties is a result of the improvement in the purity of the plastic mentioned in Example 1. Other factors that may affect this include an increase in the average molecular weight and a decrease in the molecular weight distribution. Table 4 shows the measurement results of the physical properties of the plastics recycled after microorganism treatment.

**[Table 4]**

| | Recycled Plastic | Recycled Plastic + repla1 | Recycled Plastic + repla2 | Virgin Plastic |
|---|---|---|---|---|
| Tensile strength (MPa) | 23.0 | 24.1 | 24.6 | 25.6 |
| Flexural strength (MPa) | 20.5 | 21.3 | 22.9 | 28.2 |

The extent of improvement in the physical properties of recycled plastics can be increased by optimizing the microorganism culture conditions to enhance efficiency. The microorganism culture conditions refer to pH, air injection amount, culture period, temperature, and the like. As the culture period becomes longer, the removal rate of foreign substances improves, which can lead to an improvement in physical properties (Table 5). In addition, for the same culture period, the rate of physical property improvement can be enhanced by maintaining the optimal pH level for the microorganisms through pH control (Table 6).

**[Table 5]**

| Strain | Culture Period | MFR (g/10 min) |
|---|---|---|
| Untreated | - | 29.7 |
| repla2 | 2-Day | 24.1 |
| | 7-Day | 22.1 |

**[Table 6]**

| Strain | Culture Period | pH Control | pH | MFR (g/10 min) |
|---|---|---|---|---|
| Untreated | - | - | - | 29.7 |
| repla2 | 7-Day | X | 5.0 | 25.5 |
| | 7-Day | O | 6.5 | 24.4 |
| | 7-Day | O | 6.7 | 23.6 |
| | 7-Day | O | 6.9 | 22.1 |

The improvement in the physical properties of recycled plastics can also be achieved by utilizing only the culture broth of the microorganisms. Enzymes and compounds produced by the microorganisms are present in the culture broth, which can contribute to the improvement of the purity and physical properties of recycled plastics. To confirm this, after culturing repla2 using recycled plastic as a carbon source, the cells were removed through centrifugation to obtain a supernatant. After allowing the recycled plastic to react with the supernatant, the MFR was measured at intervals of 6, 12, 24, and 48 hours. It was confirmed that the physical properties of the recycled plastic improved proportionally with time using only the culture broth without microorganisms (Table 7).

**[Table 7]**

| Experimental Group | Treatment Time | MFR (g/10 min) |
|---|---|---|
| Untreated | - | 29.7 |
| Culture broth | 6 h | 28.7 |
| Culture broth | 12 h | 28.5 |
| Culture broth | 24 h | 28.1 |
| Culture broth | 48 h | 27.9 |

### [Accession Numbers]

Name of Depositary Authority: National Institute of Agricultural Sciences
Accession Number: KACC81043BP
Date of Deposit: January 17, 2017

Name of Depositary Authority: Korean Agricultural Culture Collection (KACC), National Institute of Agricultural Sciences, Rural Development Administration
Accession Number: KACC81230BP
Date of Deposit: September 26, 2022
Name of Depositary Authority: Korean Agricultural Culture Collection (KACC), National Institute of Agricultural Sciences, Rural Development Administration
Accession Number: KACC81234BP
Date of Deposit: November 4, 2022
Name of Depositary Authority: Korean Agricultural Culture Collection (KACC), National Institute of Agricultural Sciences, Rural Development Administration
Accession Number: KACC81286BP
Date of Deposit: December 7, 2023

### [Reference Documents]

1. Trends in Biological Degradation of Plastics Utilizing Microorganisms, BRIC View 2021-T34
2. Tournier, V., Duquesne, S., Guillamot, F., Cramail, H., Taton, D., Marty, A., & André, I. (2023). Enzymes' power for plastics degradation. Chemical Reviews, 123(9), 5612-5701.
3. Gall, M., Freudenthaler, P. J., Fischer, J., & Lang, R. W. (2021). Characterization of composition and structure-property relationships of commercial post-consumer polyethylene and polypropylene recyclates. Polymers, 13(10), 1574.

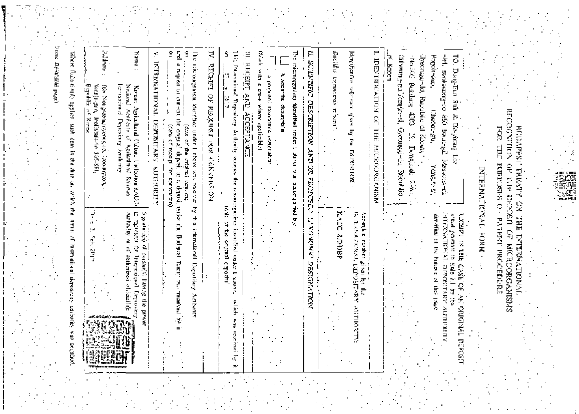

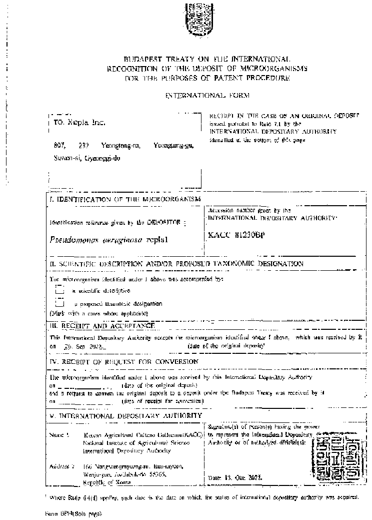

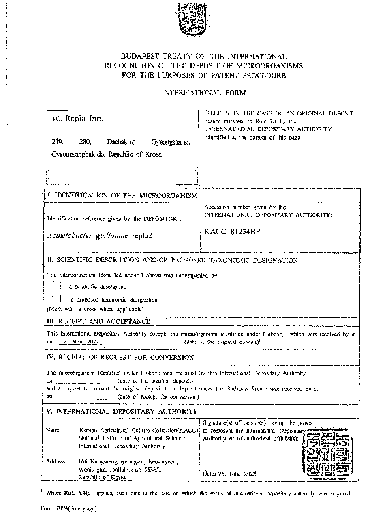

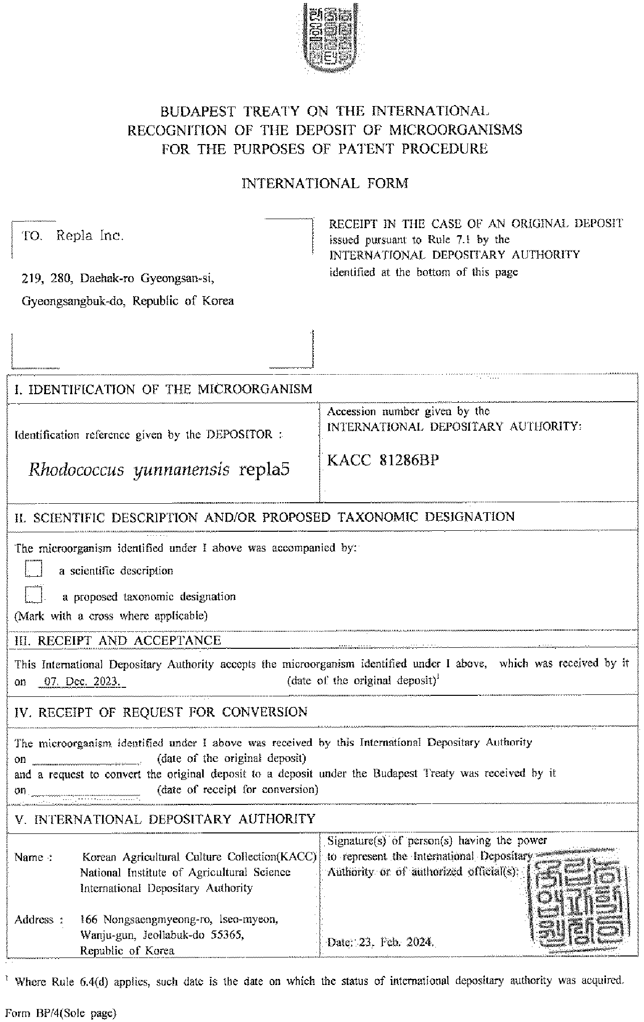

## Claims

1. A method of improving the purity of polymer waste, comprising bringing a microorganism having plastic-degrading activity, a culture broth of the microorganism, or a plastic-degrading enzyme derived from the microorganism into contact with polymer waste,
wherein the polymer is a polymer synthesized from petrochemicals or an organic polymer.

2. A method of improving physical properties of a product recycled from polymer waste, comprising bringing a microorganism having plastic-degrading activity, a culture broth of the microorganism, or a plastic-degrading enzyme derived from the microorganism into contact with polymer waste,
wherein the polymer is a polymer synthesized from petrochemicals or an organic polymer.

3. The method of claim 1 or 2, wherein the polymer synthesized from petrochemicals is one or more selected from the group consisting of polyethylene terephthalate (PET), polyvinyl chloride (PVC), polystyrene (PS), polypropylene (PP), polyurethane (PU), and polyethylene (PE).

4. The method of claim 1 or 2, wherein the organic polymer is one or more selected from the group consisting of fibers, synthetic resins, synthetic rubber, and polysaccharides.

5. The method of claim 1 or 2, wherein the contacting step is performed for 0.1 hours to 60 days.

6. The method of claim 1 or 2, wherein the microorganism having plastic-degrading activity is selected from the group consisting of a *Bacillus toyonensis* reborn strain deposited under Accession No. KACC 81043BP, a *Pseudomonas aeruginosa* repla1 strain deposited under Accession No. KACC 81230BP, an *Acinetobacter guillouiae* repla2 strain deposited under Accession No. KACC 81234BP, and a *Rhodococcus yunnanensis* repla5 strain deposited under Accession No. KACC 81286BP.

7. The method of claim 2, wherein the physical properties are thermal properties or mechanical properties of the product.

8. The method of claim 7, wherein the thermal properties of the product include a melt flow rate.

9. The method of claim 7, wherein the mechanical properties of the product include tensile strength or flexural strength.
